# EUROPEAN PATENT SPECIFICATION

(11) **EP 1 323 346 B1**
(45) Date of publication and mention of the grant of the patent: **28.06.2006**
(21) Application number: 03004479.6
(22) Date of filing: 17.01.1996
(51) Int. Cl.: A01N 63/00, A61K 39/385, A61K 39/00, A61K 47/48

(54) **Receptor specific transepithelial transport of immunogens**
Rezeptorspezifischer Transepithelealer Transport von Immunogenen
Transport épithélial spécifique de récepteurs d'immunogènes

(30) Priority: 17.01.1995 US 374159; 29.12.1995 US 578171
(43) Date of publication of application: 02.07.2003
(62) Divisional of application: 96903522.9
(73) Proprietor: THE BRIGHAM AND WOMEN'S HOSPITAL, INC., Boston, MA 02115 (US); BRANDEIS UNIVERSITY, Waltham, MA 02254 (US)
(72) Inventor: Blumberg, Richard S., Chestnut Hill, Massachusetts 02167 (US); Simister, Neil E., Wellesley, Massachusetts 02482 (US); Lencer, Wayne I., Jamaica Plain, Massachusetts 02130 (US)
(74) Representative: Killin, Stephen James

(56) References cited:
- EP-A- 0 464 533
- WO-A-86/06635
- WO-A-91/08773
- WO-A-93/20834
- KOBAYASHI K ET AL: "IDENTIFICATION OF A UNIQUE IGG FC BINDING SITE IN HUMAN INTESTINAL EPITHELIUM" JOURNAL OF IMMUNOLOGY, THE WILLIAMS AND WILKINS CO. BALTIMORE, US, vol. 143, no. 8, 15 October 1989 (1989-10-15), pages 2567-2574, XP000945300 ISSN: 0022-1767
- BOREL H ET AL: "A NOVEL TECHNIQUE TO LINK EITHER PROTEINS OR PEPTIDES TO GAMMAGLOBULIN TO CONSTRUCT TOLEROGENS" JOURNAL OF IMMUNOLOGICAL METHODS, ELSEVIER SCIENCE PUBLISHERS B.V.,AMSTERDAM, NL, vol. 126, no. 2, 9 February 1990 (1990-02-09), pages 159-168, XP002089896 ISSN: 0022-1759
- STORY C M ET AL: "A MAJOR HISTOCOMPATIBILITY COMPLEX CLASS I-LIKE FC RECEPTOR CLONED FROM HUMAN PLACENTA: POSSIBLE ROLE IN TRANSFER OF IMMUNOGLOBIN G FROM MOTHER TO FETUS" JOURNAL OF EXPERIMENTAL MEDICINE, TOKYO, JP, vol. 180, no. 6, 1 December 1994 (1994-12-01), pages 2377-2381, XP000676616 ISSN: 0022-1007
- ZHOU ET AL.: J. MOL. BIOL., vol. 332, 2003, pages 901-913,

## Description

This invention relates in general to methods and products for initiating an immune response against an antigen, and in particular relates to transepithelial delivery of antigens to provoke tolerance and immunity.

The immune-system of a mammal develops during gestation and becomes active in the late mammalian fetus. Although active, it still might be characterized as "immature" because it has not been challenged to any significant extent by antigens; the fetus is largely protected from antigens by the mother. This 'immature* immune system, however, is supplemented by the transfer of material immunoglobulin to the fetus (or in some cases to the neonate) to provide humoral immunity during the first weeks of independent life.

Rats and mice receive most maternal immunoglobulin G (IgG) as sucklings from colostrum and milk, although some is acquired prenatally. Cattle also receive IgG from colostrum. In rabbits, IgG is transported to the fetus across the yolk sac. Little is known about the transfer of IgG to the fetus or neonate in humans. Most evidence suggests that human mothers transfer humoral immunity to an offspring only before birth, although IgA transferred to a neonate via breast milk is believed to play a role in protecting the neonate against enteric infection.

The delivery of maternal IgG to the mammalian and/or neonate requires transport across an epithelial barrier which is largely impervious to macromolecules. The transport of macromolecules across such an epithelial barrier may occur by non-specific and specific receptor-mediated mechanisms. Receptor non-specific mechanisms are represented by paracellular sieving events, the efficiency of which are inversely related to the molecular weight of the transported molecule. Transport of macromolecules such as IgG across this paracellular pathway is highly inefficient. Descriptions of receptor-mediated transport of immunoglobulins through intestinal epithelial cells are limited thus far to the polymeric immunoglobulin receptor and the enterocyte receptor for IgG (a major histocompatibility complex (MHC) class I related Fc receptor). These two receptor systems differ in their specificity for immunoglobulin isotype, in their direction of immunoglobulin transport across the epithelial cell and in their tissue-specific expression. Both may play a role in molding the immature immune system.

The polymeric immunoglobulin receptor is expressed on the basolateral surfaces of enterocytes, hepatocytes and/or biliary duct epithelial cells. It transports polymeric IgA and IgM to the apical (luminal) surfaces, concentrating these immunoglobulins for antimicrobial defense and antigen exclusion.

The enterocyte receptor for IgG, which has homology to the MHC class I heavy chain and is associated with beta₂-microglobulin (β₂M), is expressed on neonatal enterocytes of the rat and mouse. IgG is transported transcellularly in a luminal to serosal direction across the intestinal epithelium of these rodent neonates. On the apical surface of the enterocyte, the Fc portion of IgG is bound to the enterocyte receptor at the relatively acidic pH of the lumen (about pH 6.0). Following transcytosis to the basolateral plasma membrane, discharge of the immunoglobulin occurs at the relatively neutral pH of the interstitial fluids (about pH 7.4). The rodent neonatal Fc receptor (FcRn) therefore could be responsible for delivery of maternal IgG to the neonate and as such may be responsible for the passive acquisition of IgG during this period

In humans, material IgG is actively transported across the placenta. The receptors responsible for this transport has been sought for many years. Several IgG-binding proteins have been isolated from placenta. FcγRII was detected in placental endothelium and FcγRIII in syncytiotrophoblasts. Both of these receptors, however, showed a relatively low affinity for monomeric IgG. Recently, the isolation from placenta of a cDNA encoding a human homolog of the rat and mouse enterocyte receptor for IgG was reported. (Story, C.M. et al., J. Exp. Med., Vol. 180:2377-2381, December 1994) The complete nucleotide and deduced amino acid sequence is reported. This Fc receptor for IgG may be responsible for the transport of maternal IgG to the human fetus (and even possibly to the neonate), as the molecule is highly homologous over its open reading frame with the rat FcRn sequence (69% nucleotide identity and 65% predicted amino acid identity). So called passive immunization in the human fetus (and possibly in the human neonate) now may become better understood.

In contrast to passive immunization which involves supplementing a host's immune system with antibodies derived from another, active immunization involves stimulation of the host's own immune system to generate in vivo the desired immune response. The most widely practiced methods of active immunization in children and adults involve injections of an immunogen, once as an initial dose and then at least once again as a booster dose. These methods suffer many serious drawbacks, including the risks associated with the use of needles that can transmit diseases such as AIDS and hepatitis. (When tolerizing a patient against an allergen, the problems are compounded in that repeated injections over a long period of time often are required). These methods also do not necessarily trigger adequately the first line of defense against many pathogens, that is, mucosal immumity. Mucous membranes line the airways, the reproductive system and the gastrointestinal tract, and this mucosal surface represents the first portal of entry for many diseases. An oral vaccine that is easy to deliver and that triggers mucosal immunity would be highly desirable.

Immunization using oral vaccines is problematic. Often little or no immune response is achieved. To enhance the immune response, antigens of interest have been coupled to carriers that are known to be strongly immunogenic. For example, researches have delivered antigens using Bacille Calmette-Guérin (BCG) as a carrier; BCG is a bacterium originally used as an oral. vaccine against tuberculosis. A problem with such carriers is that the patient will develop antibodies against the carrier itself, which can be troublesome if the carrier is used again for delivering a different antigen to the same patient. To date, no general strategy for oral vaccines has proven successful.

Immunoglobulin and portions thereof in the past have been conjugated to drugs and imaging agents to target and destroy cell populations and to extend the half-lives of certain agents: Immunotoxins are an example of such conjugates. Such conjugates, however, have never been proposed as useful for initiating an immune response.

A small body of work has focused on the tolerogenic capacity of immumoglbulins coupled to oligonucleotides or proteins characteristic of autoimmune diseases. (See PCT WO91/08773 and Borel & Borel, Journal of Immunological Methods, 126 (1990) 159-168). This work is based upon the notion that the induction of tolerance may be strongly influenced by carrier moieties and that immunoglobulin carriers appear to be strongly tolerogenic. Isologous IgG is the preferred carrier, and intravenous administration was the mode used for delivering the conjugates of IgG. Although this body of work extends for more than a decade, oral administration is mentioned only once and only for conjugates where IgA is the imunoglobilin carrier. Thus, although tolerogenic immunoglobulin conjugates are known in the art, such conjugates have never been suggested as agents for inducing a robust response against an antigen characteristic of a pathogen. (To the contrary, the art suggests that such conjugates, if anything, would tolerize a subject against a pathogen which would be highly undesirable). In addition, it never has been suggested that such conjugates would be effective tolerogens when the immunoglobulin is IgG and the mode of delivery is oral delivery.

The invention involves the discovery that antigens may be coupled to molecules that bind to the FcRn receptor, such as immunoglobulins, or portions thereof, and delivered across epithelial barriers by active transport through the enterocyte via FcRn receptors. The immunoglobulin or portion thereof binds to the FcRn receptor and acts as a carrier for the antigen as the immunoglobulin or portion thereof is transported across the epithelial barrier by FcRn mediated transport. The FcRn receptor is present in the human epithelial tissue of children and adults, and the invention therefore permits effective strategies for immunizing humans

The invention also provides: a method for modulating the immune system of a mammal An effective amount of a conjugate of an antigen and a FcRn binding partner is administered to an epithelial barrier of a mammal in need of such immune modulation. The antigen is selected from the group consisting of: an antigen that is characteristic of a pathogen, an antigen that is characteristic of an autoimmune disease, an antigen that is characteristic of an allergen and an antigen that is characteristic of a tumor. In preferred embodiments, the FcRn binding partner is non-specific IgG or a FcRn binding fragment of IgG. Most preferably the FcRn binding partner is an Fc fragment of IgG. It also is preferred that the antigen be covalently coupled to the FcRn binding partner. Preferably the conjugate is administered orally to the intestinal epithelium, in an aerosol to the lungs or intranasally. Such preparations may be nonaseptic. Supplementary potentiating agents, as described below, may be administered ia addition.

In a first aspect of the invention, there is provided a pharmaceutical preparation, for administration to an epithelial barrier of a patient to deliver a molecule across the epithelial barrier, comprising a conjugate of an FcRn binding partner coupled to said molecule, wherein the FcRn binding partner in the conjugate binds human FcRn, and wherein the preparation is formulated as a capsule, tablet, elixir or syrup for delivery to a human epithelial barrier.

In a second aspect of the invention, there is provided a pharmaceutical preparation, for administration to an epithelial barrier of a patient to deliver a molecule across the epithelial barrier, comprising a conjugate of an FcRn binding partner coupled to said molecule, wherein the FcRn binding partner in the conjugate binds human FcRn, and wherein the preparation is formulated as an aerosol for delivery to a human epithelial barrier.

The pharmaceutical preparations of the invention can include a conjugate of an antigen and a FcRN binding partner,
wherein the antigen is selected from the group consisting of: an antigen that is characteristic of a pathogen, an antigen that is characteristic of an autoimmune disease, an antigen that is characteristic of an allergen and an antigen that is characteristic of a tumor. The preferred FcRn binding partners are as described above. The conjugate is present an amount effective for modulating the immune response of a mammal. The pharmaceutical preparation also includes a pharmaceutically acceptable carrier. The pharmaceutical preparations of the invention can be formulated in unit dosage form constructed and arranged for delivery to an epithelial carrier such as for oral delivery to the intestinal epithelium, aerosol delivery to the pulmonary epithelium and intranasal delivery to the nasal epithelium. Thus tablets containing IgG (or an FcRn binding portion thereof) coupled to any of the antigens as characterized above are embraced by the presentinvention.

The foregoing pharmaceutical preparations may be delivered together with supplementary potentiating agents including adjuvants, cytokines, bioadhesives and the like. The supplementary potentiating agents themselves may be coupled to a FcRn binding partner to facilitate the delivery of such agents across the epithelial barrier. Preferred modes of administration in general include oral dosages to the intestinal epithelium, aerosols to the lungs and intranasal dosages.

Preferred embodiments of the invention are as described below or as defined in the sub-claims.

In preferred embodiments of the invention, the conjugate including the antigen crosses the epithelial barrier in an amount at least double the extent that the antigen crosses the epithelial barrier in an unconjugated form. It thus is an object of the invention to develop a mechanism for increasing the ability of an antigen to cross an epithelial barrier.

Another object of the invention is to develop a new class of orally active immunogens and toleragens.

Another object of the invention is to develop improved methods for stimulating mucosal immunity.

The invention involves the discovery that the human FcRn receptor is active in adult epithelial tissue and the discovery that FcRn binding partners such as IgG or Fc fragments can be used to transport other molecules, including antigens, across epithelial barriers. In this manner, FcRn binding partners such as IgG or an FcRn binding portion thereof can be used to deliver an antigen across an epithelial barrier to a subject's immune system, thereby initiating an immune response.

The invention is useful whenever it is desirable to enhance the delivery of an antigen across an epithelial barrier to the immune system. The invention thus may be used to deliver antigens across intestinal epithelial tissue, lung epithelial tissue and other mucosal surfaces including nasal surfaces, vaginal surfaces, colon surfaces and biliary tree surfaces. The invention may be used to modulate a subject's immune system such as by stimulating a humoral antibody response against an antigen, by stimulating T cell activity, or by stimulating tolerance to an antigen. As used herein, subject means: humans, primates, horses, cows, sheep, pigs, goats, dogs, cats, chickens and rodents.

When delivering tumor antigens, the invention may be used to treat subjects having disease amenable to immunity-mediated rejection, such as non-solid tumors or solid tumors of small size. It is also contemplated that delivery of tumor antigens by the methods described herein will be useful for treatment subsequent to removal of large solid tumors. The invention may also be used to treat subjects who are suspected of having cancer

The invention involves the formation of a conjugate of an FcRn binding partner and an antigen. By conjugate it is meant two entities bound to one another by any physiochemical means, including hydrophobic interaction between an antigen and the non-specific hydrophobic portions of an antibody molecule, antibody-antigen specific binding and covalent coupling. The nature of the preferred bonding will depend, among other things, upon the mode of administration and the pharmaceutical carriers used to deliver the conjugate to the selected epithelial barrier. For example, some bonds are not as well suited as others to withstand certain environments such as the stomach, but can be protected therefrom by delivery systems which bypass the stomach. It, of course, is important that the bond between the FcRn binding partner and the antigen be of such a nature that it does not destroy the ability of the FcRn binding partner to bind to the FcRn receptor. Such bonds are well known to those of ordinary skill in the art; examples are provided in greater detail below. The conjugate further may be formed as a fusion protein, also discussed in greater detail below.

An FcRn binding partner means any entity that can be specifically bound by the FcRn receptor with consequent active transport by the FcRn receptor of the FcRn binding partner. As mentioned above, the FcRn receptor has been isolated for several mammalian species, including humans. The sequence of the human FcRn, rat FcRn and mouse FcRn may be found in Story, C.M. et al, J. Exp. Med., vol. 180:2377-2381, December 1994. The FcRn receptor molecule now is well characterized. The FcRn receptor binds IgG (but not other immunoglobulin classes such as IgA, IgD, IgM and IgE) at a relatively lower pH, actively transports the IgG transcellularly in a luminal to serosal direction, and then releases the IgG at a relatively higher pH found in the interstitial fluids. As will be recognized by those of ordinary skill in the art, FcRn receptors can be isolated by cloning or by affinity purification using, for example, monoclonal antibodies. Such isolated FcRn receptors then can be used to identify and isolate FcRn binding partners, as described below.

FcRn binding partners include whole IgG, the Fc fragment of IgG and other fragments of IgG that include the complete binding region for the FcRn receptor. The region of the Fc portion of IgG that binds to the FcRn receptor has been described based upon X-ray crystallography (Burmeister, W.P. et al., Nature, 1994; 372:379-378.) The major contact area of Fc with the FcRn receptor is near the junction of the C_{H}2 and C_{H}3 domains. Potential contacts are residues 248, 250-257, 272, 285, 288, 290-291, 308-311 and 314 in C_{H}2 and 385-387, 428 and 433-436 in C_{H}3. these sites are distinct from those identified by subclass comparison or by site-directed mutagenesis as important for Fc binding to leukocyte FcyRI and FcγRII) The foregoing Fc - FcRn contacts are all within a single Ig heavy chain. It has been noted previously that two FcRn receptors can bind a single Fc molecule. The crystallographic data suggest that in such a complex, each FcRn molecule binds a single polypeptide of the Fc homodimer.

Given the foregoing information, those of ordinary skill in the art will readly recognize that the Fc region of IgG can be modified according to well-recognized procedures such as site-directed mutagenesis and the like to yield modified IgG or modified Fc fragments or portions thereof that will be bound by the FcRn receptor. Such modifications include modifications remote from the FcRn contact sites as well as modifications within the contact sites that preserve or even enhance binding. In addition, other binding partners can be identified and isolated. Antibodies or portions thereof specific for the FcRn receptor and capable of being transported by FcRn once bound can be identified and isolated using well established techniques. Likewise, random generated molecularly diverse libraries can be screened and molecules that are bound and transported by FcRn receptors can be isolated using conventional techniques. It is not intended that the invention be limited by the selection of any particular FcRn binding partner. Where the binding partner is IgG or a FcRn binding portion thereof, the IgG or portion thereof may be prepared according to conventional procedures as described in greater detail below.

The FcRn binding partner is conjugated with an antigen. An antigen as used herein falls into four classes: 1) antigens that are characteristic of a pathogen; 2) antigens that are characteristic of an autoimmune disease; 3) antigens that are characteristic of an allergen; and 4) antigens that are characteristic of a tumor. Antigens in general include polysaccharides, glycolipids, glycoproteins, peptides, proteins, carbohydrates and lipids from cell surfaces, cytoplasm, nuclei, mitochondria and the like.

Antigens that are characteristic of pathogens include antigens derived from viruses, bacteria, parasites or fungi. Examples of important pathogens include *Vibrio cholerae,* enterotoxigenic *Escherichia coli,* rotavirus, *Clostridium difficile, Shigella* species, *Salmonella typhi,* parainfluenza virus, influenza virus, *Streptococcus pneumoniae, Borrelia burgdorferi,* HIV, *Streptococcus mutans, Plasmodium falciparum, Staphylococcus aureus,* rabies virus and Epstein-Barr virus.

Viruses in general include but are not limited to those in the following families: picornaviridae; caliciviridae; togaviridae; flaviviridae; coronaviridae; rhabdoviridae; filoviridae: paramyxoviridae; orthomyxoviridae; bunyaviridae; arenaviridae; reoviridae; retroviridae; hepadnaviridae; parvoviridae; papovaviridae; adenoviridae; herpesviridae; and poxyviridae.

Bacteria in general include but are not limited to: *P. aeruginosa; E. coli; Klebsiella* sp.; *Serratia* sp.; *Pseudomonas* sp.; *P. cepacia; Acinetobacter sp.*; S*. epidermis, E. faecalis;* S. *pneumoniae; S. aureus*; *Haemophilus* sp.; *Neisseria* sp.; *N. meningitidis*; *Bacteroides* sp.; *Citrobacter* sp.; *Branhamella* sp.; *Salmonella* sp.; *Shigella* sp.; *S. pyogenes; Proteus* sp.; *Clostridium* sp.; *Erysipelothrix sp.; Listeria sp.; Pasteurella multocida, Streptobacillus* sp.; *Spirillum* sp.; *Fusospirocheta* sp.; *Treponema pallidum*; *Borrelia* sp.; *Actinomycetes; Mycoplasma* sp.; *Chlamydia* sp.; *Rickettsia* sp.; *Spirochaeta*; *Legionella sp.; Mycobacteria sp.; Ureoplasmo* sp.; *Streptomyces* sp.; *Trichomonas sp.;* and *P. mirabilis.*

Parasites include but are not limited to: *Plasmodium falciparum, P. vivax, P. ovale, P. malaria*; *Toxoplasma gondii; Leishmania mexicana, L tropica, L. major, L aethiopica, L. donovani*; *Trypanosoma cruzi, T. brucei; Schistosoma mansoni, S. haemaloblum, S. japonium*; *Trichinella spiralis*; *Wuchereria bancrofti*; *Brugia malayi; Entamoebo histolytica; Enterobius vermicularis ; Taenia solium, T. saginata ; Trichomonas vaginalis, T. hominis, T. tenax ; Giardia lamblia ;Cryptosporidium paruum ; Pneumocystis carinii*; *Babesia bovis, B. divergens, B. microti; Isospora belli, I. hominis*; *Dientamoeba fragilis, Onchocerca volvulus; Ascaris lumbricoides; Necator americanis; Ancylostoma duodenale*; *Strongyloides stercoralis; Capillaria philippinensis; Angiostrongylus cantonensis*; *Hymenolepis nano; Diphyllobothrium latum; Echinococcus granulosus, E. multilocularis*; *Paragonimus westermani, P. caliensis*; *Chlonorchis sinensis*; *Opisthorchis felineus, O. viverrini*; *Fasciola hepatica*; *Sarcoptes scabiei*; *Pediculus humanus; Phthiriuspubis;* and *Dermatobia hominis.*

Fungi in general include but are not limited to: *Cryptococcus neoformans*; *Blastomyces dermatitidis*; *Ajellomyces dermatitidis; Histoplasma capsulatum*; *Coccidioides immitis; Candida* species, including *C*. *albicans, C. tropicalis, C. parapsilosis,C. guilliermondii* and *C*. *krusei; Aspergillus* species, including *A. fumigatus, A. flavus* and *A. niger; Rhizopus* species; *Rhizomucor* species; *Cunninghammella* species; *Apophysomyces* species, including *A*. *saksenaea, A. mucor* and *A. absidia*; *Sporothrix schenckii*; *Paracoccidioides brasiliensis*; *Pseudallescheria boydii; Torulopsis glabrata; and Dermatophytes* species.

. Antigens that are characteristic of autoimmune disease typically will be derived from the cell surface, cytoplasm, nucleus, mitochondria and the like of mammalian tissues. Examples include antigens characteristic of uveitis (e.g. S antigen), diabetes mellitus, multiple sclerosis, systemic lupus erythematosus, Hashimoto's thyroiditis, myasthenia gravis, plimary myxoedema, thyrotoxicosis, rheumatoid arthritis, pernicious anemia, Addison's disease, scleroderina, autoimmune atrophic gastritis, premature menopause (few cases), male infertility (few cases), juvenile diabetes, Goodpasture's syndrome, pemphigus vulgaris, pemphigoid, sympathetic opthalmia, phacogenic uveitis, autoimmune haemolytic anemia, idiopatMc thrombocytopenic purpura, idiopathic leucopenia, primary biliary cirrhosis (few cases), ulcerative colitis, Sjogren's syndrome, Wegener's granulomatosis, poly/dermatomyositis, and discoid lupus erythromatosus.

Antigens that are allergens are generally proteins or glycoproteins, although allergens may also be low molecular weight allergenic haptens that induce allergy after covalently combining with a protein carrier (Remington's Pharmaceutical Sciences). Allergens include antigens derived from pollens, dust, molds, spores, dander, insects and foods. Specific examples include the urushiols (pentadecylcatechol or heptadecylcatechol) of *Toxicodendron* species such as poison ivy, poison oak and poison sumac, and the sesquiterpenoid lactones of ragweed and related plants.

Antigens that are characteristic of tumor antigens typically will be derived from the cell surface, cytoplasm, nucleus, organelles and the like of cells of tumor tissue. Examples include antigens characteristic of tumor proteins, including proteins encoded by mutated oncogenes; viral proteins associated with tumors; and tumor mucins and glycolipids. Tumors include, but are not limited to, those from the following sites of cancer and types of cancer: lip, nasopharynx, pharynx and oral cavity, esophagus, stomach, colon, rectum, liver, gall bladder, biliary tree, pancreas, larynx, lung and bronchus, melanoma of skin, breast, cervix, uteri, uterus, ovary, bladder, kidney, brain and other parts of the nervous system, thyroid, prostate, testes, Hodgkin's disease, non-Hodgkin's lymphoma, multiple myeloma and leukemia. Viral proteins associated with tumors would be those from the classes of viruses noted above. Antigens characteristic of tumors may be proteins not usually expressed by a tumor precursor cell, or may be a protein which is normally expressed in a tumor precursor cell, but having a mutati'on-chamctcristic of a tumor. An antigen characteristic of a tumor may be a mutant variant of the normal proteins having an altered activity or subcellular distribution. Mutations of genes giving rise to tumor antigens, in addition to those specified above, may be in the coding region, 5' or 3' noncoding regions, or introns of a gene, and may be the result of point mutations, frameshifts, deletions, additions, duplications, chromosomal rearrangements and the like. One of ordinary skill in the art is familiar with the broad variety of alterations to normal gene structure and expression which gives rise to tumor antigens. Specific examples of tumor antigens include: proteins such as Ig-idiotype of B cell lymphoma, mutant cyclin-dependent kinase 4 of melanoma, Panel-17 (gp100) of melanoma, MART-1 (Melan-A) of melanoma, p15 protein of melanoma, tyrosinase of melanoma, MAGE 1, 2 and 3 of melanoma, thyroid medullary, small cell lung cancer, colon and/or bronchial squamous cell cancer, BAGE of bladder, melanoma, breast, and squamous cell carcinoma, gp75 of melanoma, oncofetal antigen of melanoma; carbohydrate/lipids such as muc1 mucin of breast, pancreas, and ovarian cancer, GM2 and GD2 gangliosides of melanoma; oncogenes such as mutant p53 of carcinoma, mutant ras of colon cancer and HER-2/*neu* proto-oncogene of breast carcinoma; viral products such as human papillomavirus proteins of squamous cell cancers of cervix and esophagus. It is also contemplated that proteinaceous tumor antigens may be presented by HLA molecules as specific peptides derived from the whole protein. Metabolic processing of proteins to yield antigenic peptides is well known in the art; for example see U.S. patent 5,342,774 (Boon et al.). The present method thus encompasses delivery of antigenic peptides and such peptides in a larger polypeptide or whole protein which give rise to antigenic peptides. Delivery of antigenic peptides or proteins may give rise to humoral or cellular immunity.

Generally, subjects can receive an effective amount of the tumor antigen, and/or peptide derived therefrom by one or more of the methods detailed below. Initial doses can be followed by booster doses, following immunization protocols standard in the art. Delivery of tumor antigens thus may stimulate proliferation of cytolytic T lymphocytes.

In the cases of protein and peptide antige, covalent linking to an FcRn partner is intended to include linkage by peptide bonds in a single polypeptide chain. Established methods (Sambrook et al., Molecular Cloning; A Laboratory Manual, Cold Spring Harbor Press; Cold Spring Harbor, NY 1989) would be used to engineer DNA encoding a fusion protein comprised of the antigenic peptide or protein and an FcRn partner. This DNA would be placed in an expression vector and introduced into bacterial or cukaryotic cells by established methods. The fusion protein would be purified from the cells or from the culture medium by established methods.

When administered, the conjugates of the present invention are administered in pharmaceutically acceptable preparations. Such preparations may routinely contain pbarmaceutically acceptable concentrations of salt, buffering agents, preservatives, compatible carriers, supplementary immune potentiating agents such as adjuvants and cytokines, and optionaly other therapeutic agents. Thus, "cocktails" including the conjugates and the agents are contenmplated. The agents themselves may be conjugated to FcRn binding partners to enhance delivery of the agents across the epithelial barries.

The conjugates of the invention may be administered per se (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof and are not excluded from the scope of the invention. Such pharmaceutically acceptable salts include, but are not limited to, those prepared from the following acids: hydrochloric, hydrobromic, sulphuric, nitric, phosphoric, maleic, acetic, salicyclic, p-toluene sulphonic, tartaric, citric, methane sulphonic, formic, malonic, succinic, naphthaene-2-sulphonic, and benzene sulphonic. Also, pharmaceutically acceptable salts can be prepared as alkaline metal or alkaline carth salts, such as sodium, potassium or calciuin salts of the carboxylic acid group.

Suitable buffering agents include: acetic acid and a salt (1-2% W/V); citric acid and a salt (1-3% W/V); boric acid and a salt (0.5-2.5% W/V); sodium bicarbonate (0.5-1.0% W/V); and phosphoric acid and a salt (0.8-2% W/V). Suitable preservatives include benzalkonium chloride (0.003-0.03% W/V); chlorobutanol (0.3-0.9% W/V); parabens (0.01-0.25% W/V) and thimerosal (0.004-0.02% W/V).

The term "carrier" as used herein, and described more fully below, means one or more solid or liquid filler, dilutants or encapsulating substances which are suitable for administration to a human or other mammal. The "carrier" may be an organic or inorganic ingredient, natural or synthetic, with which the active ingredient is combined to facilitate administration.

The components of the pharmaceutical compositions are capable of being comingled with the conjugates of the present invention, and with each other, in a manner such that there is no interaction which would substantially impair the desired pharmaceutical efficiency. The components of oral drug formulations include diluents, binders, lubricants, glidants, disintegrants, coloring agents and flavoring agents. Encapsulating substances for the preparation of enteric-coated oral formulations include cellulose acetate phthalate, polyvinyl acetate phthalate, hydroxypropyl methylcellulose phthalate and methacrylic acid ester copolymers. Solid oral formulations such as capsules or tablets are preferred. Elixirs and syrups also are well known oral formulations. The components of aerosol formulations include solubilized active ingredients, antioxidants, solvent blends and propellants for solution formulations, and micronized and suspended active ingredients, dispersing agents and propellants for suspension formulations. The oral, aerosol and nasal formulations of the invention can be distinguished from injectable preparations of the prior art because such formulations may be nonaseptic, whereas injectable preparations must be aseptic.

The term "adjuvant" is intended to include any substance which is incorporated into or administered simultaneously with the conjugates of the invention and which nonspecifically potentiates the immune response in the subject. Adjuvants include aluminum compounds, e.g., gels, aluminum hydroxide and aluminum phosphate, and Freund's complete or incomplete adjuvant (in which the conjugate is incorporated in the aqueous phase of a stabilized water in paraffin oil emulsion). The paraffin oil may be replaced with different types of oils, e.g., squalene or peanut oil. Other materials with adjuvant properties include BCG (attenuated *Mycobacterium tuberculosis),* calcium phosphate, levamisole, isoprinosine, polyanions (e.g., poly A:U) leutinan, pertussis toxin, cholera toxin, lipid A, saponins and peptides, e.g. muramyl dipeptide. Rare earth salts, e.g., lanthanum and cerium, may also be used as adjuvants. The amount of adjuvants depends on the subject and the particular conjugate used and can be readily determined by one skilled in the art without undue experimentation.

Other supplementary immune potentiating agents, such as cytokines, may be delivered in conjunction with the conjugates of the invention. The cytokines contemplated are those that will enhance the beneficial effects that result from administering the immunomodulators according to the invention. Cytokines are factors that support the growth and matmiation of cells, including lymphocytes. It is believed that the addition of cytokines will augment cytokine activity stimulated in vivo by carrying out the methods of the invention. The preferred cytokines are interleukin (IL)-1, IL-2, gamma-interferon and tumor necrosis factor α. Other useful cytokines are believed to be IL-3, IL-4, IL-5, IL-6, IL-7, IL-8, IL-9, IL-10, IL-11, IL-12, IL-13, erythropoietin, leukemia inhibitory factor, oncostatin-M, ciliary neurotrophic factor, growth hormone, prolactin, CD40-ligand, CD27-ligand, CD30-ligand, alpha-interfaron, beta-interferon, and tumor necrosis factor-β. Other cytokines known to modulate T-cell activity in a manner likely to be useful according to the invention are colony stimulating factors and growth factors including granulocyte and/or macrophage stimulating factors (GM-CSF, G-CSF and CSF-1) and platelet derived, epidermal, insulin-like, transforming and fibroblast growth factors. The selection of the particular cytokines will depend upon the particular modulation of the immune system that is desired. The activity of cytokines on particular cell types is known to those of ordinary skill in the art.

The precise amounts of the foregoing cytokines used in the invention will depend upon a variety of factors, including the conjugate selected, the dose and dose-timing selected, the mode of administration and the characteristics of the subject. The precise amounts selected can be determined without undue experimentation, particularly since a threshold amount will be any amount which will enhance the desired immune response. Thus, it is believed that nanogram to milligram amounts are useful, depending upon the mode of delivery, but that nanogram to microgram amounts are likely to be most useful because physiological levels of cytokines are correspondingly low.

The preparations of the invention are administered in effective amounts. An effective amount is that amount of a conjugate that will alone, or together with further doses, stimulate an immune response as desired. This may involve the stimulation of a humoral antibody response resulting in an increase in antibody titer in serum, improved mucosal immunity, a clonal expansion of cytotoxic T lymphocytes or tolerance to an antigen, including a self antigen. It is believed that doses ranging from 1 nanogram/kilogram to 100 milligrams/kilogram, depending upon the mode of administration, will be effective. The preferred range is believed to be between about 500 nanograms and 500 microgramslkilogram, and most preferably between 1 microgram and 100 micrograms/kilogram. The absolute amount will depend upon a variety of factors, including the conjugate selected, the immune modulation desired, whether the administration is in a single or multiple doses, and individual patient parameters including age, physical condition, size and weight. For treatment of a subject with a tumor, the size, type, location and metastases of the tumor may be factored in when determining the amount of conjugate to administer. These factors are well known to those of ordinary skill in the art and can be addressed with no more than routine experimentation.

A variety of administration routes are available. The particular mode selected will depend, of course, upon the particular conjugate selected, the particular condition being treated and the dosage required for therapeutic efficacy. The methods of this invention, generally speaking, involve delivering the conjugates of the invention to an epithelial surface. Preferred modes of administration are oral, intrapulmonary, intrabiliary and intranasal.

Compositions may be conveniently presented in unit dosage form and may be prepared by any of the methods well known in the art of pharmacy. All methods include the step of bringing the conjugate into association with a carrier which constitutes one or more accessory ingredients. In general, the compositions are prepared by uniformly and intimately bringing the conjugate into association with a liquid carrier, a finely divided solid carrier, or both, and then, if necessary, shaping the produce.

Other delivery systems can include time-release, delayed release or sustained release delivery systems. Such systems can avoid repeated administrations of the conjugates of the invention, increasing convenience to the subject and the physician. Many types of release/delivery systems are available and known to those of ordinary skill in the art. They include polymer based systems such as polylactic and polyglycolic acid, polyanhidrides and polycaprolactone; wax coatings, compressed tablets using conventional binders and excipients, and the like. Bioadhesive polymer systems to enhance delivery of a material to the intestinal epithelium are known and described in published PCT application WO 93/21906. Capsules for delivering agents to the intestinal epithelium also are described in published PCT application WO 93/19660.

### EXAMPLES

### Materials

### Abbreviations

BSA, bovine serum albumin; cDNA, complementary deoxyribonucleic acid; CT-B, cholera toxin B subunit; DMEM, Dulbecco's modified Eagle's medium; DMSO, dimethyl sulfoxide; DOC, desoxycholate; ECL, enhanced chemiluminescence; ELISA, enzyme linked immunosorbant assay; HBSS, Hanks' balanced salt solution without calcium or magnesium; HEPES, N-[2-hydroxyethyl]piperazine-N'-[2-ethanesulfonic acid]; hGH, human growth hormone; IEC, intestinal epithelial cells; KI, potassium iodide; MHC, major histocompatibility complex; NaOH, sodium hydroxide; NH₄Cl, ammonium chloride; NHS-rhodamine, N-hydroxysuccinimidyl-rhodamine; RNA, ribonucleic acid; RT-PCR, reverse transcriptase-polymerase chain reaction; SATA, N-succinimidyl S-acetylthioacetate; SDS-PAGE, sodium dodecyl sulfate-polyacrylamide gel electrophoresis; sulfo-LC-SPDP, sulfosuccinimidyl 6-[3-(2-pyridyldithio)propionamide] hexanoate; sulfo-NHS-biotin, sulfosuccinimidobiotin; sulfo-SMCC, sulfosuccinimidyl 4-(N-meleimidomethyl) cyclo-hexane-1-carboxylate.

### Chemicals

cDNA Cycle Kits was purchased from Invitrogen (San Diego, CA). Taq polymerase was purchased from Perkin-Elmer Cetus (Norwalk, CT). CircumVent^{™} Kits were purchased from New England Biolabs (Beverly, MA). Radionuclides and radioactive chemicals were purchased from DuPont/NEN (Boston, MA). HBSS and DMEM were purchased from GIBCO/Life Technologies (Gaithersburg, MD). RPMI 1640 was purchased fromCellogro (Hemdon, VA). L-glutamine was purchased from Cellgro. Protein A-Sepharose was purchased from Pharmacia Biotech (Piscataway, NJ). Streptavidin-horseradish peroxidase, sulfo-LC-SPDP, sulfo-NHS-biotin, sulfo-SMCC, SATA and immobilized ficin were purchased from Pierce (Rockford, IL). Balb/c mice were purchased from Charles River Laboratories (Wilmington, MA). ECL kits were purchased from Amersham (Arlington Heights, IL).

Plasmin, AvidChrom-protein A, protein G-Sepharose, BSA, cholera toxin B subunit, anti-hGH anu-bodi, and all other chemicals were purchased from Sigma (St. Louis, MO)

### Example 1: Expression of FcRn mRNA in Human intestinal

### Epithelial Primary Cells and CellLinens

Total RNA was extracted from adult human enterocytes by standard methodology well known in the art (Sambrook et al., ibid.). One microgram of RNA from each cell type was used as a template to prepare the cDNA substrate for reverse transcriptase-polymerase chain reaction (RT-PCR) using a cDNA Cycle Kit (Invitrogen, San Diego, CA). Thirty cycles of PCR were performed on the cDNA using Taq polymerase (Perkin-Elmer Cetus, Norwalk, CT) according to the manufacturer's instructions using primers TGCTGGGCTGTGAACTG and CGCTTTTAGCAGTCGGAA. The PCR cycle conditions were: denaturation at 94°C for one minute, annealing at 55°C for two minutes and extension at 72°C for three minutes. Amplification products were resolved by electrophoresis on a 1.5% agarose gel and visualized by ethidium bromide staining, which showed the presence of the expected approximately 800 base pair amplification product in all samples except the adult colonic epithelial cells. To confirm the identity of the RT-PCR amplification product, the DNA band was excised from the agarose gel, subcloned into pCR II (Invitrogen, San Diego, CA) and sequenced using a Prism^{™} dye-deoxy terminator cycle sequencing kit (Applied Biosystems, Foster City, CA) using primers from both vector and human FcRn sequence. Reaction products were analyzed on an Applied Biosystems sequencer. The sequence of the amplification products exactly matched the FcRn gene sequence, confirming the identity of the expressed gene.

### Example 2: Detection of FcRn mRNA by Northern Blot

To confirm the expression of FcRn in human intestinal epithelial cells and cell lines, a Northern blot was prepared using the RNA samples prepared as described in Example 1 from adult human enterocytes, and from two human adenocarcinoma cell lines of colonic origin, Caco-2 and HT-29. The RNA samples were resolved by formaldehyde/agarose gel electrophoresis and transferred to a nylon membrane by standard procedures (Sambrook et al., Molecular Cloning: A laboratory Manual, Cold Spring Harbor Press, Cold Spring Harbor, NY 1989). The membrane was probed using a ³²P-radiolabeled 120 base pair probe from the 3' untranslated region of FcRn by standard methods. Autoradiograms of the Northern blot demonstrated the presence of the 1.5 kilobase hFcRn transcript in the enterocytes and both cell lines. Therefore, the expression of FcRn in human adult intestinal epithelial cells and cell lines was demonstrated by two different methods of RNA detection.

### Example 3 : Labeling and Immunoprecipitation of the

### MHC-Class I Related Fc Receptor (FcRn) from Intestinal Epithelial Cells

The expression of FcRn in human intestinal epithelial cells was confirmed by immunoprecipitation of the protein. Caco-2 cells were labeled metabolically using ³³S.methionine (DuPont/NEN, Boston, MA) and proteins were extracted by methods well known in the art (Harlow and Lane, Antibodies: A Laboratory Manual). A polyclonal rabbit anti-rat MHC class I related FcR heavy chain specific antiserum bound to protein-A-sepharose was used to immunoprecipitate FcRn from the cell extracts using standard methods (FcRn can be purified by well established methods, Simister and Rees 1985, European J. Immunology, 15:733-8, and used to inununize rats followed by collection of serum, Harlow and Lane, supra.). Immunoprecipitates were resolved by SDS-PAGE and visualized by autoradiography. A 48 kilodalton FcRn protein was observed, confirming expression observed at the RNA level.

### Example 4: Expression of FcRn Protein on the Cell Surface of Human Intestinal Epithelial Cells

About 3 X 10⁷ HT-29 inti-sdnal epithelial cells were detached from tissue culture plates by nonenzymatic methods and were washed four times with ice cold Hanks' balanced silt solution containing no calcium or magnesium (HBSS-, GIBCO/Life Technologies, Gaithersburg, MD). To label cell surface proteins, the washed cells were incubated twice for 20 minutes with 1.5 ml of 0.5 mg/ml sulfo-NHS-biotin (Pierce, Rockford, IL) in DMSO. Labeled cells were washed five times with 50 mM NH₄Cl, incubated 20 minutes with 10 ml of RPMI 1640 (Cellgro, Heindon, VA) containing 1 mM L-glutamine (Mediatech, Washington, DC), and washed four times with HBSS-. The cells were lysed, then precleared overnight with protein A-Sepharose beads (Pharmacia Biotech, Piscataway, NJ) using standard techniques well known in the art. SDS and desoxycholic acid (DOC) were added to the supernatants to final concentrations of 0.1% and 0.5%, respectively. Lysates were precleared with normal rabbit serum and immunoprecipitated with polyclonal rabbit anti-rat MHC class I related FcR antibody by methods well known in the art. Immunoprecipitates were resolved by SDS-PAGE and transferred to nitrocellulose membranes. The nitrocellulose membrane was processed for incubation with 1:10,000 diluted streptavidin-horseradish peroxidase (Pierce, Rockford, IL), as recommended by the manufacturer. The membrane was then processed for detection of bound horseradish peroxidase using an ECL kit (Amersham, Arlington Heights, IL). Light emitted by cleavage of the cherniluminescent substrate was detected by exposure of the membrane to light-sensitive film. The film exposures showed that FcRn was expressed on the surface of HT-29 intestinal epithelial cells.

### Example 5: Functional Activity of Human FcRn on the Cell

### Surfaceof Intestinal Epithelial Cells

To show that the FcRn expressed on the cell surface of intestinal epithelial cells was functional, Caco-2 cells and human adult jejunal intestinal epithelial cells (IECs) were tested for the ability to bind Fc fragment of antibody. Caco-2 and jejunal IECs were distributed to microcentrifuge tubes (2X10⁶ cells per tube) and pelleted at 2000 rpm for 2-3 minutes at 4°C. Cell pellets were washed once in DMEM containing 20mM HEPES, pH 6.0 or pH 8.0 at 4°C and resuspended in 0.2 ml of the same medium. The cell suspensious were transferred to 12 well plates for assay. ¹²⁵I-Fc fragment (200ng/ml, 4 X 10⁻⁹ M) in DMEM containing 20 mM HEPES 1.0 mM KI and 0.1% fish gelatin, pH 6.0 or pH 8.0 with or without 0.5 mg/ml unlabeled human IgG (3.3 X 10⁻⁶M) was added to each well. The cells were allowed to bind IgG or Fc at 37°C for two hours in a 5% CO₂ humidified atmosphere. Cells were transferred to microcentrifuge tubes and pelleted at 2000 rpm for 2-3 minutes at 4°C. Unsound I-Fc was removed by washing the cell pellets once with cold DMEM containing 20 mM HEPES, pH 6.0 or pH 8.0 at 4°C. Cells were disrupted in 0.5 ml 0.1 M NaOH and the resulting solution transferred to scintillation vials. ¹²⁵I was quantified using a CliniGamma 1272 gamma counter (LKB Wallac, Piscataway, NJ). Both Caco-2 cells and human adult jejunum IECs specifically bound ¹²⁵I-Fc at pH 6.0 but not at pH 8.0, demonstrating functional pH-dependent binding as observed for rat neonatal FcRn and cloned human FcRn (Story et al., J. Exp. Med. 180: 2377-2381; December 1994).

### Example 6: Preparation of Human Immunoglobulin G

Non-specific purified immunoglobulin G from human, mouse, rat, goat, pig, cow, and other species may be purchased from commercial vendors such as Sigma Chemical Co., Pierce Chemical, HyClone Laboratories, ICN Biomedicals and Organon Teknika-Cappel

Immunoglobulin G also may be isolated by ammonium sulfate precipitation of blood serum. The protein precipitate is further fractionated by ion exchange chromatography or gel filtration chromatography, to isolate substantially purified non-specific IgG. By non-specific IgG it is meant that no single specificity within the antibody population or pool is dominant.

Immunoglobulin G also may be purified from blood serum by adsorption to protein A attached to a solid support such as protein A-Sepharose (Pharmacia), AvidChrom-Protein A (Sigma), or protein G-Sepharose (Sigma). Other methods of purification of IgG are well known to persons skilled in the art and may be used for the purpose of isolation of non-specific IgG.

### Example 7: Preparation of Human Immunoglobulin G Fc Fragment

To prepare the Fc fragments of human IgG, IgG isolated as in Example 6 is subjected to digestion with immobilized papain (Pierce) according to the manufacturer's recommended protocol. Other proteases that digest IgG to produce intact Fc fragments that can bind to Fc receptors, e.g. plasmin (Sigma) or immobilized ficin (Pierce), are known to skilled artisans and may be used to prepare Fc fragments. The digested immunoglobulin then is incubated with an affinity matrix such as protein A-Sepharose or protein G-Sepharose. Non-binding portions of IgG are eluted from the affinity matrix by extensive washing in batch or column format. Fc fragments of IgG then are eluted by addition of a buffer that is incompatible with Fc-adsorbant binding. Other methodologies effective in the purification of Fc fragments also may be employed.

### Example 8: Comucation of Compounds to Human Immunoglobulin Fc Fragments

To deliver compounds via the FcRn transport mechanism, such compounds can be coupled to whole IgG or Fc fragments. The chemistry of cross-linking and effective reagents for such purposes are well known in the art. The nature of the crosslinking reagent used to conjugate whole IgG or Fc fragments and the compound to be delivered is not restricted by the invention. Any crosslinking agent may be used provided that a) the activity of the compound is retained, and b) binding by the FcRn of the Fc portion of the conjugate is not adversely affected.

An example of an effective one-step crosslinking of Fc and a compound is oxidation of Fc with sodium periodate in sodium phosphate buffer for 30 minutes at room temperature, followed by overnight incubation at 4 °C with the compound to be conjugated. Conjugation also may be performed by derivatizing both the compound and Fc fragments with sulfosuccinimidyl 6-t3-(2-pyridyldithio)propionamide] hexanoate (sulfo-LC-SPDP, Pierce) for 18 hours at room temperature. Conjugates also may be prepared by derivatizing Fc fragments and the desired compound to be delivered with different crosslinking reagents that will subsequently form a covalent linkage. An example of this reaction is derivatization of Fc fragments with sulfosuccinimidyl 4-(N-maleimidomethyl) cyclo-hexane-1-carboxylate (Sulfo-SMCC, Pierce) and the compound to be conjugated to Fc is thiolated with N-succinimidyl S-acetylthioacetate (SATA). The derivatized components are purified free of crosslinker and combined at room temperature for one hour to allow crosslinking. Other crosslinking reagents comprising aldehyde, imide, cyano, halogen, carboxyl, activated carboxyl, anhydride and maleimide functional groups are known to persons of ordinary skill in the art and also may be used for conjugation of compounds to Fc fragments. The choice of cross-linking reagent will, of course. depend on the nature of the compound desired to be conjugated to Fc. The crosslinking reagents described above are effective for protein-protein conjugations. If the compound to be conjugated is a carbohydrate or has a carbohydrate moiety, then heterobifunctional crosslinking reagents such as ABH, M₂C₂H, MPBH and PDPH are useful for conjugation with a proteinaceous FcRn binding molecule (Pierce Chemical Co., Rockford, IL). Another method of conjugating proteins and carbohydrates is disclosed by Brumeanu et al. (Genetic Engineering News, October 1, 1995, p.16). If the compound to be conjugated is a lipid or has a lipid moiety which is convenient as a site of conjugation for the FcRn binding molecule, then crosslinkers such as SPDP, SMPB and derivatives thereof may be used (Pierce Chemical Co., Rockford, IL). It is also possible to conjugate any molecule which is to be delivered by noncovalent means. One convenient way for achieving noncovalent conjugation is to raise antibodies to the compound to be delivered, such as monoclonal antibodies, by methods well known in the art, and select a monoclonal antibody having the correct Fc region and desired antigen binding properties. The antigen to be delivered is then prebound to the monoclonal antibody carrier. In all of the above crosslinking reactions it is important to purify the derivatized compounds free of crosslinking reagent. It is important also to purify the final conjugate substantially free of unconjugated reactants. Purification may be achieved by affinity, gel filtration or ion exchange chromatography based on the properties of either component of the conjugate. A particularly preferred method is an initial affinity purification step using protein A-Sepharose to retain Fc and Fc-compound conjugates, followed by gel filtration or ion exchange chromatography based on the mass, size or charge of the Fc conjugate. The initial step of this purification scheme ensures that the conjugate will bind to FcRn which is an essential requirement of the invention.

### Example 9: IgG-Facilitated Delivery of Foreign Antigen

### Across the Intestinal Epithelial Barrier

To test the ability of Fc binding partner-antigen conjugates to be transported across epithelial barriers, foreign antigens are conjugated to IgG molecules for administration to mice. A convenient foreign antigen is the fluorescent dye rhodamine, since it may be visualized in frozen semi-thin sections of intestinal epithelium. Rhodamine is covalently linked to non-specific mouse IgG, prepared as described in Example 6, cholera toxin B subunit (Sigma) and ovalbumin (Sigma) by incubation with succinyl-rhodamine (Molecular Probes, Eugene, OR) as recommended by the manufacturer. The IgG-rhodamine conjugate is purified by protein G-Sepharose affinity chromatography. After dialysis to remove unconjugated succinyl-rhodamine, cholera toxin B (CT-B)-rhodamine and ovalbumin-rhodamine conjugates are purified by gel filtrations or ion exchange chromatography. Fractions of the eluate are assayed for the presence of conjugates by determining fluorescence. Functional binding of the IgG and CT-B subunit conjugates may be tested by binding to FcRn and ganglioside GM1, respectively. Cholera toxin B-rhodamine and ovalbumin-rhodamine serve as positive and negative controls, respectively.

Balb/c mice are administered 0.2 nanomoles of the three rhodamine cojugates described above, with or without 0.2 nanomoles unlabeled cholera toxin as a nomspecific adjuvant, by intragastric administration in the presence of 75 micromoles NaHCO₃ and 20 mg/ml soybean trypsin inhibitor to inhibit gastric degradation. After 6 hours the mice are sacrificed and intestine is removed, frozen and processed for semi-thin sectioning. Sections of the intestinal epithelium are illuminated on a fluorescent microscope and examined for intracellular fluorescence. The presence of fluorescence in intestinal epithelial cells of mice fed IgG-rhodamine indicates that the IgG conjugates are effectively transported in an apical to basolateral direction across the intestinal epithelial barrier. FcRn is capable of transporting immunogens as conjugates with FcRn binding partners.

### Example 10: Mouse Mucosal Immune Response to

### Orally Delivered Antigen-IgG Conjugate Via FcRn-Mediated Transcytosis

Transgenic mice homozygous for deletion of β₂-microglobulin (a critical component of Fc-receptor function) and their normal wild-type litter mates are used for studies of generation of a mucosal immune response. If rhodamine-IgG elicits a mucosal immune response by binding to apical membrane Fc receptors, a positive immune response should be found in wild-type but not β₂-microglobulin "knockout" mice. In contrast, rhodamine-cholera toxin B subunit (CT-B) should elicit a positive immune response in both wild type and "knockout" mice as transcytosis of CT-B across the epithelial barrier is not dependent on binding to apical membrane Fc receptors. Rhodamine-ovalbumin does not enter transcytotic vesicles (but may enter intestinal epithelia by fluid phase endocytosis) and should not elicit an immune response in any mice.

Three groups of wild type and β₂-microglobulin knockout mice are orally immunized with the three rhodamine conjugates described in Example 9. Parallel experiments are conducted with the addition of 0.2 nanomoles of cholera toxin as non-specific adjuvant. Equimolar quantities of the rhodamine conjugates are administered intragastrically. The mice are "immunized" by this method every ten days for a total of three times. Two weeks after the third oral immunization the mice are sacrificed and the rhodamine-specific immune response its determined by ELISA on gut secretions and serum by standard methodology. Anti-rhodamine serum irrirnunoglobulins are most evident in the wild type mice fed rhodamine conjugates of CT-B and IgG. Knockout mice lacking β₂-microglobulin generate a mucosal immune response to rhodamine-CT-B but not to rhodamine-IgG, indicating that receptor-mediated transcytosis plays an essential role in the mucosal immune response. The control rhodamine-ovalbumin conjugate elicits little or no immune response in either the wild type or the β₂-microglobulin knockout mice.

## Claims

1. A pharmaceutical preparation, for administration to an epithelial barrier of a patient to deliver a molecule across the epithelial barrier, comprising a conjugate of an FcRn binding partner coupled to said molecule, wherein the FcRn binding partner in the conjugate binds human FcRn, and wherein the preparation is formulated as a capsule, tablet, elixir or syrup for delivery to a human epithelial barrier.

2. A pharmaceutical preparation, for administration to an epithelial barrier of a patient to deliver a molecule across the epithelial barrier, comprising a conjugate of an FcRn binding partner coupled to said molecule, wherein the FcRn binding partner in the conjugate binds human FcRn, and wherein the preparation is formulated as an aerosol for delivery to a human epithelial barrier.

3. A pharmaceutical preparation as claimed in claim 1 or claim 2, wherein the molecule is an antigen or a therapeutic agent.

4. A pharmaceutical preparation as claimed in claim 1 or claim 2, wherein the molecule is a cytokine.

5. A pharmaceutical preparation as claimed in claim 4, wherein the cytokine is an interleukin, a growth factor, an interferon, or a tumor necrosis factor.

6. A pharmaceutical preparation as claimed in claim 5, wherein the cytokine is alpha-interferon.

7. A pharmaceutical preparation as claimed in claim 5, wherein the cytokine is beta-interferon.

8. A pharmaceutical preparation as claimed in claim 4, wherein the cytokine is erythropoietin.

9. A pharmaceutical preparation as claimed in claim 1 or claim 2, wherein the molecule is an antigen characteristic of a tumor.

10. A pharmaceutical preparation as claimed in claim 1 or claim 2, wherein the molecule is an antigen characteristic of a pathogen.

11. A pharmaceutical preparation as claimed in claim 1 or claim 2, wherein the molecule is an antigen characteristic of an allergen.

12. A pharmaceutical preparation as claimed in claim 1 or claim 2, wherein the molecule is an antigen characteristic of an autoimmune disease.

13. A pharmaceutical preparation as claimed in any of claims 1-12, wherein the FcRn binding partner is nonspecific IgG or an FcRn binding fragment of IgG.

14. A pharmaceutical preparation as claimed in any of claims 1-12, wherein the FcRn binding partner is an Fc fragment of IgG.

15. A pharmaceutical preparation as claimed in any of claims 1-12, wherein the FcRn binding partner is covalently coupled to the molecule.

16. A pharmaceutical preparation as claimed in any of claims 1 or 3 to 15, wherein the preparation is constructed and arranged for delivery to an epithelial barrier as an oral dosage.

17. A pharmaceutical preparation as claimed in any of claims 2 to 15, wherein the preparation is constructed and arranged for delivery to an epithelial barrier as an aerosol dosage.

18. A pharmaceutical preparation as claimed in any of claims 2 to 15, wherein the preparation is constructed and arranged for delivery to an epithelial barrier as an intranasal dosage.

## Patentansprüche

1. Pharmazeutische Zubereitung für die Verabreichung an eine Epithelbarriere eines Patienten für die Abgabe eines Moleküls durch die Epithelbarriere, umfassend ein Konjugat eines FcRn-Bindungspartners, der an das Molekül gekoppelt ist, wobei der FcRn-Bindungspartner in dem Konjugat menschliches FcRn bindet und wobei die Zubereitung als Kapsel, Tablette, Elixier oder Sirup für die Abgabe an eine menschliche Epithelbarriere formuliert ist.

2. Pharmazeutische Zubereitung für die Verabreichung an eine Epithelbarriere eines Patienten für die Abgabe eines Moleküls durch die Epithelbarriere, umfassend ein Konjugat eines FcRn-Bindungspartners, der an das Molekül gekoppelt ist, wobei der FcRn-Bindungspartner in dem Konjugat menschliches FcRn bindet und wobei die Zubereitung als Aerosol für die Abgabe an eine menschliche Epithelbarriere formuliert ist.

3. Pharmazeutische Zubereitung nach Anspruch 1 oder Anspruch 2, wobei das Molekül ein Antigen oder ein therapeutisches Mittel ist.

4. Pharmazeutische Zubereitung nach Anspruch 1 oder Anspruch 2, wobei das Molekül ein Zytokin ist.

5. Pharmazeutische Zubereitung nach Anspruch 4, wobei das Zytokin ein Interleukin, ein Wachstumsfaktor, ein Interferon oder ein Tumornekrosefaktor ist.

6. Pharmazeutische Zubereitung nach Anspruch 5, wobei das Zytokin Alphainterferon ist.

7. Pharmazeutische Zubereitung nach Anspruch 5, wobei das Zytokin Betainterferon ist.

8. Pharmazeutische Zubereitung nach Anspruch 4, wobei das Zytokin Erythropoietin ist.

9. Pharmazeutische Zubereitung nach Anspruch 1 oder Anspruch 2, wobei das Molekül ein für einen Tumor kennzeichnendes Antigen ist.

10. Pharmazeutische Zubereitung nach Anspruch 1 oder Anspruch 2, wobei das Molekül ein für einen Krankheitserreger kennzeichnendes Antigen ist.

11. Pharmazeutische Zubereitung nach Anspruch 1 oder Anspruch 2, wobei das Molekül ein für ein Allergen kennzeichnendes Antigen ist.

12. Pharmazeutische Zubereitung nach Anspruch 1 oder Anspruch 2, wobei das Molekül ein für eine Autoimmunkrankheit kennzeichnendes Antigen ist.

13. Pharmazeutische Zubereitung nach einem der Ansprüche 1 bis 12, wobei der FcRn-Bindungspartner ein nichtspezifisches IgG oder ein FcRn-Bindungsfragment von IgG ist.

14. Pharmazeutische Zubereitung nach einem der Ansprüche 1 bis 12, wobei der FcRn-Bindungspartner ein Fc-Fragment von IgG ist.

15. Pharmazeutische Zubereitung nach einem der Ansprüche 1 bis 12, wobei der FcRn-Bindungspartner kovalent an das Molekül gekoppelt ist.

16. Pharmazeutische Zubereitung nach einem der Ansprüche 1 oder 3 bis 15, wobei die Zubereitung für die Abgabe an eine Epithelbarriere als orale Dosierung konstruiert und angeordnet ist.

17. Pharmazeutische Zubereitung nach einem der Ansprüche 2 bis 15, wobei die Zubereitung für die Abgabe an eine Epithelbarriere als Aerosoldosierung konstruiert und angeordnet ist.

18. Pharmazeutische Zubereitung nach einem der Ansprüche 2 bis 15, wobei die Zubereitung für die Abgabe an eine Epithelbarriere als intranasale Dosierung konstruiert und angeordnet ist.

## Revendications

1. Préparation pharmaceutique destinée à être administrée sur une barrière épithéliale chez un patient pour faire passer une molécule au travers de cette barrière épithéliale, qui comprend un conjugué composé d'un partenaire de liaison aux récepteurs FcRn couplé à ladite molécule, le partenaire de liaison aux récepteurs FcRn du conjugué se liant aux récepteurs FcRn humains et ladite préparation se présentant sous la forme d'une gélule, d'un comprimé, d'un élixir ou d'un sirop pour administration sur une barrière épithéliale humaine.

2. Préparation pharmaceutique destinée à être administrée sur une barrière épithéliale chez un patient pour faire passer une molécule au travers de cette barrière épithéliale, qui comprend un conjugué composé d'un partenaire de liaison aux récepteurs FcRn couplé à ladite molécule, le partenaire de liaison aux récepteurs FcRn du conjugué se liant aux récepteurs FcRn humains et ladite préparation se présentant sous la forme d'un aérosol pour administration sur une barrière épithéliale humaine.

3. Préparation pharmaceutique selon la revendication 1 ou la revendication 2, où ladite molécule est un antigène ou un agent thérapeutique.

4. Préparation pharmaceutique selon la revendication 1 ou la revendication 2, où ladite molécule est une cytokine.

5. Préparation pharmaceutique selon la revendication 4, où ladite cytokine est une interleukine, un facteur de croissance, un interféron ou un facteur de nécrose tumorale.

6. Préparation pharmaceutique selon la revendication 5, où ladite cytokine est un interféron alpha.

7. Préparation pharmaceutique selon la revendication 5, où ladite cytokine est un interféron bêta.

8. Préparation pharmaceutique selon la revendication 4, où ladite cytokine est une érythropoïétine.

9. Préparation pharmaceutique selon la revendication 1 ou la revendication 2, où ladite molécule est un antigène caractéristique d'un type de tumeur.

10. Préparation pharmaceutique selon la revendication 1 ou la revendication 2, où ladite molécule est un antigène caractéristique d'un agent pathogène.

11. Préparation pharmaceutique selon la revendication 1 ou la revendication 2, où ladite molécule est un antigène caractéristique d'un allergène.

12. Préparation pharmaceutique selon la revendication 1 ou la revendication 2, où ladite molécule est un antigène caractéristique d'une maladie auto-immune.

13. Préparation pharmaceutique selon l'une quelconque des revendications 1-12, où ledit partenaire de liaison aux récepteurs FcRn est une IgG non spécifique ou un fragment d'IgG qui se lie aux récepteurs FcRn.

14. Préparation pharmaceutique selon l'une quelconque des revendications 1-12, où ledit partenaire de liaison aux récepteurs FcRn est le fragment Fc d'une IgG.

15. Préparation pharmaceutique selon l'une quelconque des revendications 1-12, où ledit partenaire de liaison aux récepteurs FcRn est couplé de façon covalente à la molécule.

16. Préparation pharmaceutique selon l'une quelconque des revendications 1 ou 3 à 15, qui est élaborée et agencée pour être administrée par voie orale sur une barrière épithéliale.

17. Préparation pharmaceutique selon l'une quelconque des revendications 2 à 15, qui est élaborée et agencée pour être administrée en aérosol sur une barrière épithéliale.

18. Préparation pharmaceutique selon l'une quelconque des revendications 2 à 15, qui est élaborée et agencée pour être administrée par voie intranasale sur une barrière épithéliale.
